Europäisches Patentamt

(19) European Patent Office

Office européen des brevets

(11) Publication number: **0 097 432**
**A2**

# EUROPEAN PATENT APPLICATION

(12)

(21) Application number: 83303010.9

(22) Date of filing: 25.05.83

(51) Int. Cl.³: **A 61 M 1/03**

(30) Priority: 18.06.82 CA 405499

(43) Date of publication of application:
04.01.84 Bulletin 84/1

(84) Designated Contracting States:
DE FR GB IT

(71) Applicant: Dadson, Joseph E.
60 Littleleaf Drive
Scarborough Ontario M1B 1Z3(CA)

(71) Applicant: Agarwal, Mahesh C.
44 Raymerville Drive
Markham Ontario(CA)

(72) Inventor: Dadson, Joseph E.
60 Littleleaf Drive
Scarborough Ontario M1B 1Z3(CA)

(72) Inventor: Agarwal, Mahesh C.
44 Raymerville Drive
Markham Ontario(CA)

(74) Representative: Bond, Bentley George et al,
Haseltine Lake & Co. 28 Southampton Buildings
Chancery Lane
London WC2A 1AT(GB)

(54) Method and apparatus for peritoneal dialysis.

(57) There is provided a dialysis apparatus which includes first means for supplying dialysis fluid, second means for receiving, heating and weighing dialysis fluid, third means for receiving and weighing a quantity of dialysis fluid, a catheter, and structure which supports the first means above the second means, the second means above the catheter, and the third means below the catheter. A first flow path interconnects the first and second means, while a second flow path interconnects the second means in the catheter. A third flow path interconnects the catheter in the third means. Valve means are provided for selectively (i) blocking the first and third paths while leaving the second path open, (ii) blocking the second and third paths while leaving the first path open, and (iii) blocking the second path while leaving the first and third paths open. A timer/computer is provided which is able to maintain mode (i) until a preselected reduction in weight occurs at the second means, following which it switches to and maintains mode (ii), following which it switches to and maintains mode (iii).

./...

FIG. 2

# METHOD AND APPARATUS FOR PERITONEAL DIALYSIS

This invention relates to a method and apparatus for performing peritoneal dialysis, capable of use in both the hospital and home environment.

## BACKGROUND OF THIS INVENTION

Peritoneal dialysis can be used for correcting the following medical disorders:

1. Acute and chronic renal failure
2. Severe water retention
3. Electrolyte disorders and
4. Drug intoxication (acute poisoning).

The normal function of the kidneys is to excrete metabolic waste products from the body. The kidneys also regulate accurately the amount and composition of body fluids. The kidneys perform important endocrine functions as well, some of which are blood pressure regulation, bone marrow function, and bone composition and turnover.

In kidney failure, the above functions are affected to varying degrees. When kidney failure is mild or moderate, some of the resulting abnormalities can be corrected or ameliorated by drugs or dietary measures. When the kidney failure is severe however, artificial kidney function becomes necessary to maintain life. Artificial kidney (dialysis) treatment cannot totally compensate for the patient's own kidneys. Dialysis primarily substitutes for the lost excretory function and helps regulate fluid, electrolyte and acid-base balance.

There are two types of artificial kidney treatment, namely hemodialysis and peritoneal dialysis.

Hemodialysis is a direct treatment of the blood using an extra-corporeal system with an artificial membrane (kidney), while peritoneal dialysis uses the principles of osmosis and diffusion across the peritoneal membrane to indirectly remove toxic substances from the blood, and thereby correct certain electrolyte and fluid imbalances. As a result, extra-corporeal hemodialysis is used when rapid and efficient dialysis is necessary to preserve the life of the patient in cases of severe renal failure or drug intoxication.

Hemodialysis is technically more demanding than peritoneal dialysis, and this along with other medical reasons has led to increasing use of the relatively simple peritoneal operation for (a) acute and chronic renal failure, (b) severe water retention, (c) electrolyte disorders and (d) drug intoxication (acute poisoning).

Since the early seventies when the introduction of reliable clinical sterile techniques reduced the rate of infection in peritoneal dialysis treatment, the number of end stage renal failure patients being maintained on peritoneal dialysis has steadily grown to a very sizeable population. The need to provide a simple, less infectious and less costly treatment for the patient has led to many forms of peritoneal dialysis techniques, the major and widely used ones being the following:

1.     Automatic Peritoneal Dialysis, which uses complicated fluid proportioning machines.

2.     Intermittent Peritoneal Dialysis (I.P.D.), which uses Cyclers and commercially prepackaged dialysate.

3.     Continuous Ambulatory Peritoneal Dialysis (C.A.P.D.), which is a completely manual operation using commercially prepackaged dialysate.

4.     Continuous Cycling Peritoneal Dialysis (C.C.P.D.), which is a technique utilizing the advantages of I.P.D. (Cycler) and C.A.P.D.

(manual) operations, and is the newest technique.

The present apparatus is intended to be used for I.P.D. and C.C.P.D. However, as C.C.P.D. is the latest peritoneal dialysis technique for treating end stage renal failure patients, the following discussion focuses mainly on C.C.P.D.

Some of the major advantages of C.A.P.D. are the continuous long hours of treatment, the relatively high middle molecules clearances, reasonable freedom of mobility, lower operational cost, lower initial capital equipment cost, and procedural simplicity. Amongst its obvious disadvantages are the inconvenience of multiple daily exchanges, exchanges taking place at unusual locations (i.e. work areas), the low patients' compliance, and very high incidence of peritonitis. The high infection rate may be due to many factors. However, the obvious suspected causes of the peritonitis are the large number of disconnections and connections required, and the lack of a well organized clean area dedicated to the carrying out of exchange procedures. The C.A.P.D. exchange procedures normally result in interruptions in daily activities, significantly reduce a patient's productive working time, and produce patient fatigue. Patient fatigue also can lead to compromises in the sterile procedures which are very important in peritoneal dialysis. Such compromise can often lead to infection. The high cost of treating C.A.P.D. infection and the resulting rapid deterioration of the peritonium cavity due to high infection rates, has made C.A.P.D. less attractive than it might have been.

On the other hand I.P.D., using the automated cycler, has been shown to produce less infection because of the smaller number of non-sterile breaks. However, the intermittent dialysis procedures of I.P.D., the lack of patient mobility during treatment, and the high cost of dialysate used, has limited the growth of I.P.D.

The C.C.P.D. technique combines the advantages of I.P.D. and C.A.P.D., and thus far is proving to be highly successful. However, the same old operational set-up methods of I.P.D. are being used for C.C.P.D. As such, there is little or no economic operational savings for C.C.P.D. over I.P.D. The saving on solution cost is counteracted by the high cost of the disposable tubing.

GENERAL DESCRIPTION OF THIS INVENTION

It is an aspect of this invention to provide a peritoneal dialysis method and apparatus which, when used for C.C.P.D., will provide increased simplicity and more economical operation, and which, when used for I.P.D., will also provide financial savings and additionally provide services which are more than what the current automated cyclers are providing.

Accordingly, this invention provides a dialysis apparatus comprising first means for supplying dialysis fluid, second means for receiving, heating and weighing dialysis fluid, third means for receiving and weighing a quantity of dialysis fluid, and a catheter. Structure is provided to support the first means above the second means, the second means above the catheter and the third means below the catheter. A first fluid flow path is defined to interconnect the first and second means, while a second fluid flow path is provided to interconnect the second means and the catheter. A third fluid flow path interconnects the catheter and the third means. The apparatus includes valve means for selectively (i) blocking the first and third paths while leaving the second path open, (ii) blocking the second and third paths while leaving the first path open, and (iii) blocking the second path while leaving the first and third paths open. The apparatus further includes a timer/computer means which maintains mode (i) until a preselected reduction in weight occurs at the second means, then switches to and maintains mode (ii), then switches to and maintains mode (iii).

This invention further provides a method of peritoneal dialysis utilizing a dialysis apparatus comprising first means for supplying dialysis fluid, second means for receiving, heating and weighing dialysis fluid, third means for receiving and weighing a quantity of dialysis fluid, and a catheter connected to the peritoneal cavity of a patient. Structure supports the first means above the second means, the second means above the catheter and the third means below the catheter. Further means defines a first fluid flow path interconnecting the first and second means, a second fluid flow path interconnecting the second means and the catheter, and a third fluid flow path interconnecting the catheter and the third means. The method involves the following steps in rotational sequence. The first and third paths are blocked while leaving the second path open until a preselected reduction in weight occurs at the second means, whereby a quantity of dialysis fluid corresponding to the reduction in weight passes by gravity along the second flow path into the peritoneal cavity of the patient. · Then, the second and third paths are blocked and the first path is left open, whereby to initiate replenishment of fresh dialysis fluid in the second means from the first means along the first flow path, while maintaining dialysis fluid in the peritoneal cavity of the patient. Finally, the second path is blocked while the first and third paths are left open, whereby fresh dialysis fluid continues to pass along the first path from the first means to the second means, and simultaneously dialysis fluid from the peritoneal cavity of the patient drains along the catheter and the third path into the third means. Mode (i) constitutes the fill mode for the patient; mode (ii) constitutes the dwell mode for the patient; and mode (iii) constitutes the drain mode for the patient.

This invention further provides a dialysis apparatus which includes first means for supplying dialysis fluid, the first means including a plurality of prepackaged, sterile bags of dialysis fluid, second means for receiving, heating and weighing dialysis fluid, the portion of the second means which receives the dialysis fluid being constituted by a further one of such prepackaged, sterile bags of dialysis fluid, third means

6

0097432

for receiving and weighing a quantity of dialysis fluid, and a catheter. Structure supports the first means above said second means, the second means above the catheter and the third means below the catheter. A first fluid flow path is defined to interconnect the first and second means, while a second fluid flow path is defined to interconnect the second means and the catheter. A third fluid flow path is provided to interconnect the catheter and the third means. The apparatus includes valve means for selectively (i) blocking the first and third paths while leaving the second path open, (ii) blocking the second and third paths while leaving the first path open, and (iii) blocking the second·path while leaving the first and third paths open. A timer/computer is provided to cycle modes (i), (ii) and (iii) in a continuous manner.

Finally, this invention provides a method of peritoneal dialysis utilizing a dialysis apparatus comprising first means for supplying dialysis fluid, said first means comprising a plurality of prepackaged, sterile bags of dialysis fluid, second means for receiving, heating and weighing dialysis fluid, the portion of the second means which receives the dialysis fluid being constituted by a further one of such prepackaged, sterile bags of dialysis fluid, third means for receiving and weighing a quantity of dialysis fluid, a catheter connected to the peritoneal cavity of a patient, and structure supporting the first means above the second means, the second means above the catheter, and the third means below the catheter. Further means are provided to define a first flow path interconnecting the first and second means, a second flow path interconnecting the second means and the catheter, and a third flow path interconnecting the catheter and the third means. The method includes the following basic steps in rotational sequence. First and third paths are blocked while leaving the second path open, so that a quantity of dialysis fluid passes by gravity along the second flow path into the peritoneal cavity of the patient. Then the second and third paths are blocked while leaving the first path open, thus initiating replenishment of fresh dialysis fluid in the second means from the first means along the first flow path, and maintaining dialysis fluid in the peritoneal cavity of the patient. Then, the second path is blocked while the first and third paths are opened, whereby fresh dialysis fluid continues to pass along the first path from the first means to the second means, and simultaneously fluid from the peritoneal cavity of the patient drains along the catheter and the third path into the third means.

## GENERAL DESCRIPTION OF THE DRAWINGS

Four embodiments of this invention are illustrated in the accompanying drawings, in which like numerals denote like parts throughout the several views, and in which:

Figure 1 is a schematic view of a prior art peritoneal dialysis apparatus;

Figure 2 is a schematic view of a first embodiment of the peritoneal dialysis apparatus of this invention;

Figure 3 is a schematic view of a second embodiment of the peritoneal dialysis apparatus of this invention;

Figure 4 is a schematic view of a third embodiment of the peritoneal dialysis apparatus of this invention; and

Figure 5 is a schematic view of a fourth embodiment of the peritoneal dialysis apparatus of this invention.

## DETAILED DESCRIPTION OF THE DRAWINGS

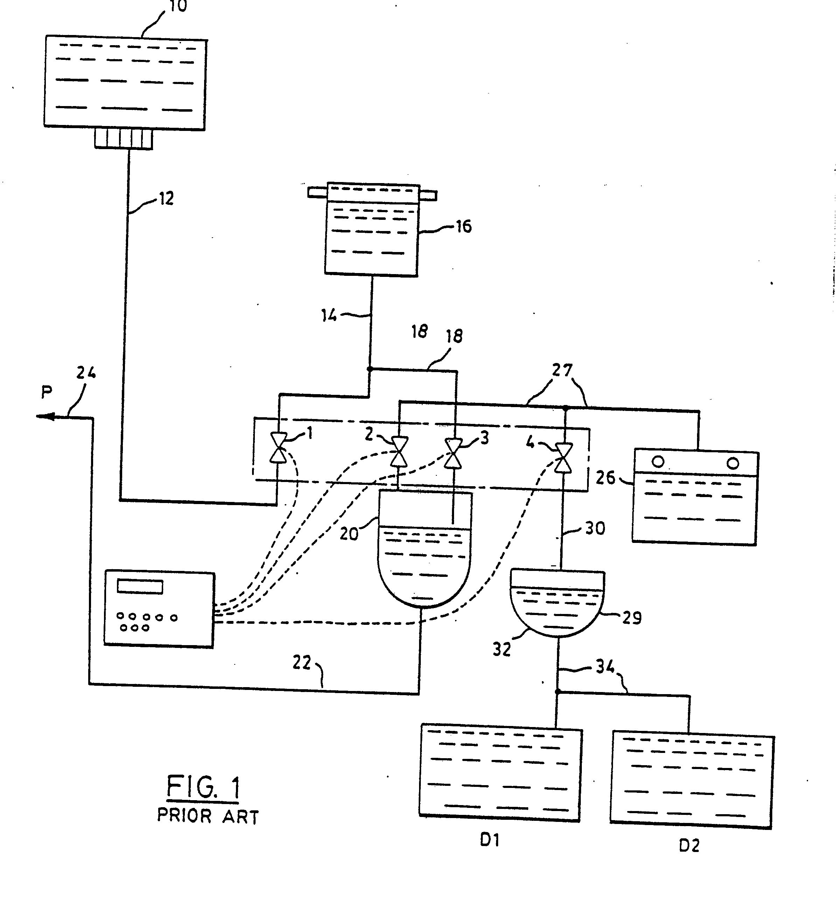

Attention is first directed to Figure 1, which shows schematically a peritoneal dialysis apparatus based on that disclosed in our own prior U.S. Patent No. 4,096,859, issued June 27, 1978, and entitled "Apparatus for Peritoneal Dialysis".

In Figure 1, cold dialysate fluid is supplied to the system from a container 10 which may in actual practice be constituted by one or more prepackaged, sterile plastic bags filled with dialysate fluid. Structure (not shown) is provided to hang the container 10 in the uppermost position by comparison with the other containers. A fluid flow path which includes tube 12 and tube 14 is adapted to direct cold dialysate fluid from container 10 to container 16 which is equipped with conventional means for warming the dialysate to an appropriate temperature to be admitted to the peritoneal cavity of a patient. From the container 16, the warmed dialysate flows to the patient P along tube 14, through tube 18, through a drip chamber 20, and along a tube 22 to a catheter 24. This constitutes a "fill" mode for the patient.

When sufficient dialysate has entered the peritoneal cavity of the patient, a "dwell" mode is initiated, in which the dialysate is maintained in the peritoneal cavity for a predetermined period of time. Thereafter, in a "drain" mode, the dialysate in the peritoneal cavity, plus excess fluid from the patient, flow out to a waste bag 26, moving along a path which includes the catheter 24, the tube 22, drip chamber 20, and a tube 27. Finally, the fluid in the waste bag 26 flows out along a portion of the tube 27, a branching tube 30, a drip chamber 32, and a tube network 34 into drain containers $D_1$, $D_2$ ...$D_n$.

The pole or other structure which holds the container 10 allows the same to be lowered for loading and raised for normal operation. This moving mechanism adds complexity and extra weight to the cart of this prior art structure.

In this prior art structure, as seen in Figure 1, the containers 16, 26, and $D_1$ ...$D_n$ are all expensive sterile bags which need to be provided for each separate operation.

It is an aspect of some embodiments of this invention to eliminate the need for these expensive sterile bags, leaving only the low inexpensive network of sterile tubing to be provided for the operation of the apparatus.

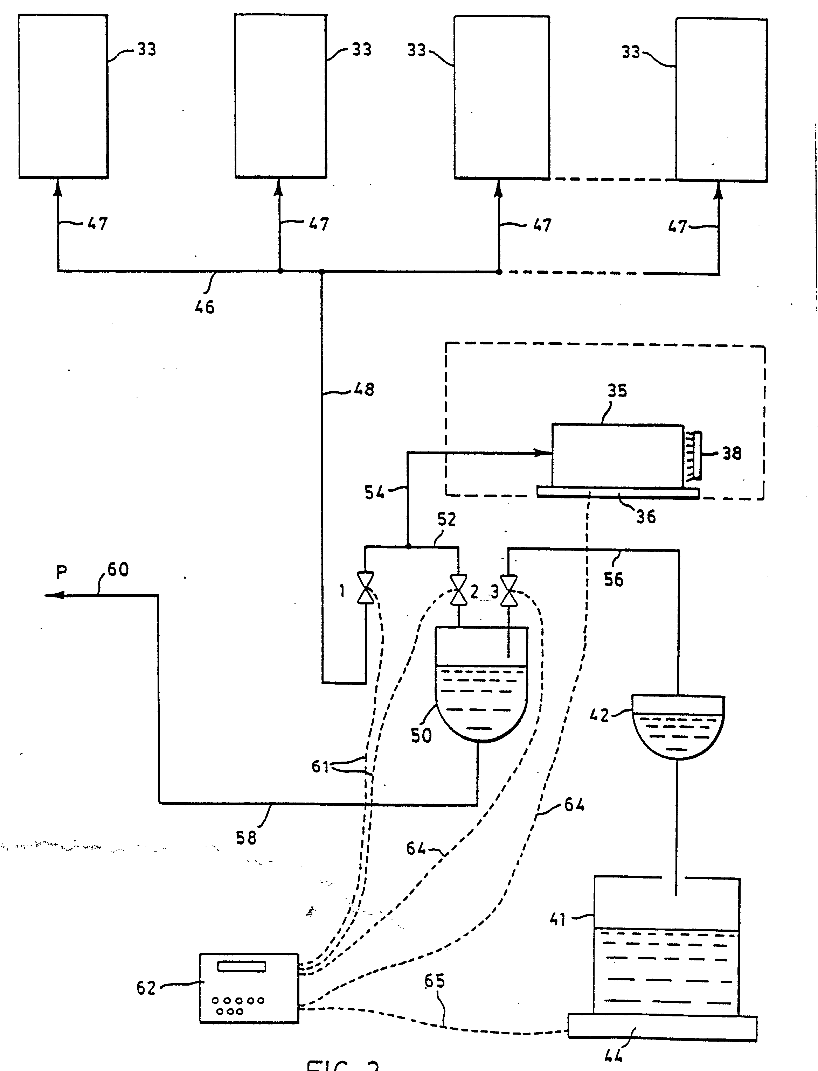

Attention is now directed to Figure 2, which shows the first embodiment of this invention. The rectangles identified by the numeral 33 represent the commercially prepackaged sterile dialysate bags, which are normally collapsible and disposable. One of the prepackaged solution bags 35 is positioned on a special electronic scale 36, and is positioned to receive heat from a conventional heater shown schematically at 38 in Figure 2. This eliminates the necessity for the prior art "special" dialysate warming bag 16 seen in Figure 1.

Instead of the complex waste disposal arrangement of the prior art shown at bottom right in Figure 1, a permanent, reusable container 41 is employed. The reusable container 41, which is downstream of a drip chamber 42, is placed on a further electronic scale 44 which is able to keep track of the amount of solution drained from the peritoneal cavity of a patient. The reusable container 41, in combination with the electronic scale 44, replaces the waste bag 26 and the series of drain bags $D_1$, $D_2$ ...$D_n$.

Another important difference between this embodiment and the prior art apparatus shown in Figure 1 is the use of only three independent valves identified as 1, 2 and 3 in Figure 2. These valves may be of any standard or conventional kind, and typically would be independently operated occluding cams having a structure similar to that disclosed in our earlier U.S. Patent 4,096,859.

To complete the description of the apparatus in Figure 2, it will be seen that a trunk line 46 receives dialysate from the containers 33 along branch lines 47, and feeds dialysate into a tube 48 which communicates with a drip chamber 50 through the valve 1, a tube 52 and the valve 2. The tube 48 also communicates with the container 35 through a tube 54. From the drip chamber 50, dialysate can flow through valve 3 along a tube 56 to the drip chamber 42, and thence to the reusable container 41. Also from the drip chamber 50 (the bottom thereof) extends a tube 58 connecting to the catheter 60 which communicates in turn with the peritoneal cavity of the patient P.

In the method of operation, of which a description follows, the two electronic scales 36 and 44 permit a computation of the amount of fluid removed from the patient during treatment. The excess of fluid removed over fluid entering is called the ultrafiltration, and the amount of the ultrafiltration is used for blood pressure and physiological controls. The computation is done by a timer/computer 62 which is connected to the scales by lines 64 and 65, shown broken in Figure 2.

This ability to automatically compute ultrafiltration eliminates or reduces the current method of periodic weighing and blood pressure measurements on the patient. Along lines 64, the timer/computer 62 also controls the valves 1, 2 and 3 in Figure 2.

The Figure 2 apparatus is adapted to operate in three distinct modes, called FILL, DWELL and DRAIN.

1.  FILL: For the FILL mode, the first and third valves are closed, while the second valve 2 is left open. This prevents dialysate from the containers 33 from passing to the container 35, and it halts any flow through the line 56. What is permitted is a flow of dialysate from the container 35, along tubes 54 and 52, through the drip chamber 50, and along the tube 58 and catheter 60 to the peritoneal cavity of the patient.

Rather than run the FILL mode for a preselected period of time, as in the prior art, the present apparatus maintains the FILL mode until a preselected reduction in weight occurs in the container 35, as detected by the timer/computer 62 as it monitors the instantaneous readings of the electronic scale 36. The amount of fluid to be allowed into the peritoneal cavity of the patient is preselected, and thus this amount can be tailored to the body weight and size of the patient. Because the FILL operates on a weight basis rather than the conventional time basis, the operation of the machine can be tailored to individuals of widely differing weight and body size. For example, children may take as little as 100 mg of dialysate, whereas an adult may require 2 kgm of dialysate. The apparatus herein disclosed can adapt to either of these extremes without any modification. The timer/computer 62 also has the capability of generating an alarm signal if during the fill mode the rate of reduction of weight of the container 35 is below a predetermined threshold.

2.DWELL: The DWELL operates on a time basis which is preselectable from zero to a number of hours in increments of one minute. For the DWELL mode, the second and third valves are closed, while the first valve remains open. While the first valve is opened, dialysate can begin to pass from the containers 33 to the container 35, to replenish dialysate in the latter.

3.DRAIN: For the DRAIN mode, the second path is blocked, while the first and third paths are open. This permits dialysate to continue to pass from the containers 33 to the container 35, if required, and also allows used dialysate and excess fluid from the peritoneal cavity of the patient to pass along the catheter 60, the tube 58, through the drip chamber 50 in the reverse sense, past the valve 3, along tube 56, through drip chamber 42 and finally to the reusable container 41. Using the second electronic scale 44, the timer/computer is able to keep track of the flow of dialysate out of the peritoneal cavity of the patient. This is done by comparing the weight of the quantity of fluid received in the reusable container 41 during a DRAIN mode with the preselected reduction in weight of the container 35 during the previous FILL mode. When the former exceeds the latter, the timer/computer can calculate the difference and display it. Because problems can arise if the ultrafiltration is too great, the timer/computer 62 is adapted to generate an alarm signal if the ultrafiltration calculated as above described exceeds a predetermined amount. The timer/computer 62 has means for allowing the operator to manually select this predetermined ceiling on the ultrafiltration. The timer/computer 62 is also adapted to generate an alarm signal if, during the DRAIN mode, the rate at which the rate of collected fluid in the reusable container 41 increases is not above a predetermined threshold rate, or if outflow from the patient does not equal the previous inflow. The latter can be expressed more precisely by saying that an alarm signal is generated if, by the end of the preselected period of time for the drain mode, the preselected reduction in weight of the container 35 during the preceding FILL mode is greater than the weight of the total quantity of fluid collected in the reusable container 41.

Because of these operational conditions in the FILL and DRAIN modes, the machine can measure the dialysate inflow and outflow, calculate the ultrafiltration and display the same. Therefore, the operator does not have to set maximum fluid intake volume and readjust the corresponding minimum alarm drain volume. The timer/computer is also adapted to generate an alarm and to halt the filling of the patient when extremely cold temperature (frozen solution) dialysate is used. This prevents the use of hypertonic dialysate, which will cause hypotension. A severe hypotension can cause fatal complications.

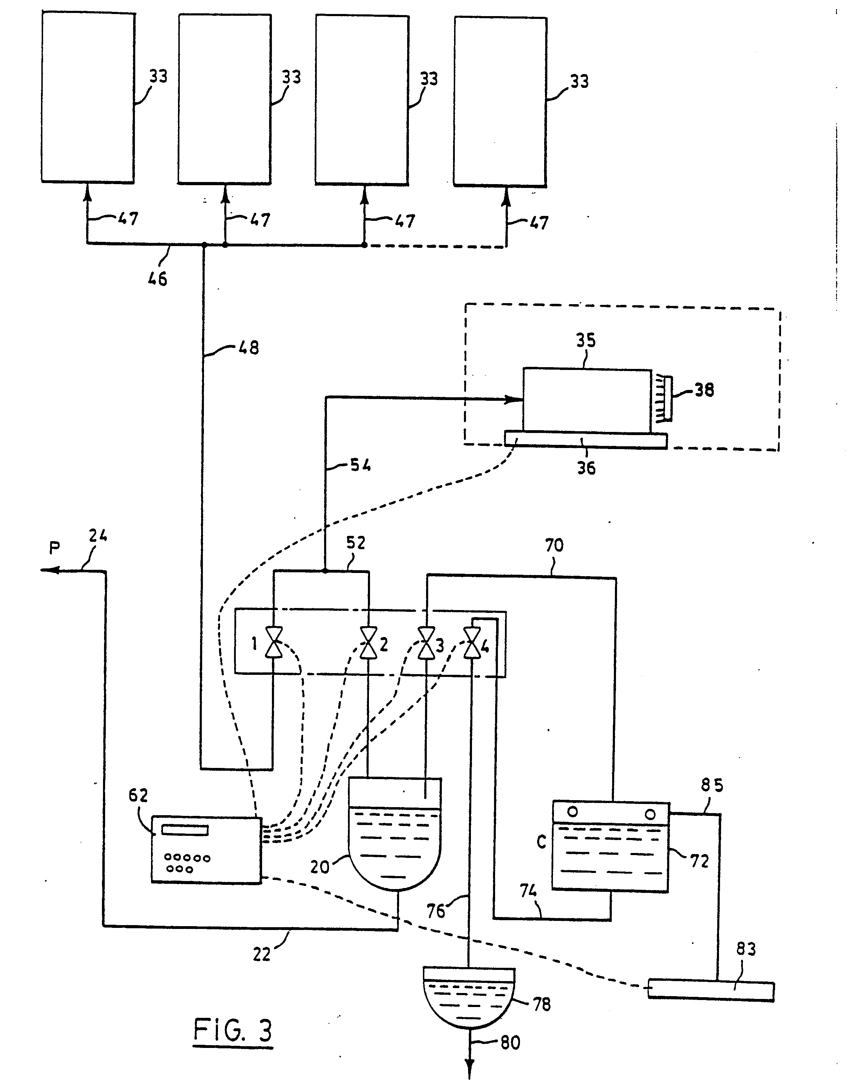

Attention is now directed to Figure 3, which shows the second embodiment of this invention. In Figure 3, all portions identical to equivalent portions in Figure 2 bear the same numbers. The primary difference between the Figure 3 embodiment and the Figure 2 embodiment is the presence of a fourth valve, and a different arrangement for receiving the waste dialysate from the patient. Instead of the tube 56 shown in Figure 2, the Figure 3 embodiment has a tube 70 extending from the valve 3 and entering a waste bag 72 at the top. From the waste bag 72, a drain line 74 passes through the fourth valve (valve 4) and finally along a line 76 to a drip chamber 78 (for isolation purposes), thence to a final drain along the line 80. The line 80 may also pass to drainage bags similar to the bags $D_1$, $D_2$ ...$D_n$ shown in Figure 1. An electronic scale 83 is adapted to monitor the weight of the waste bag 72 through an arm means shown schematically at 85. The effect is the same as in Figure 2, in which the electronic scale 44 monitors the accumulation of waste dialysate and additional liquids from the peritoneal cavity of the patient.

In the Figure 3 embodiment, the fourth fluid flow path represented by the tubes 74, 76 and 80, is open in the FILL and DWELL mode, and is closed in the DRAIN mode.

Aside from these differences, the approach and operation of the Figure 3 embodiment is substantially the same as that of the Figure 2 embodiment.

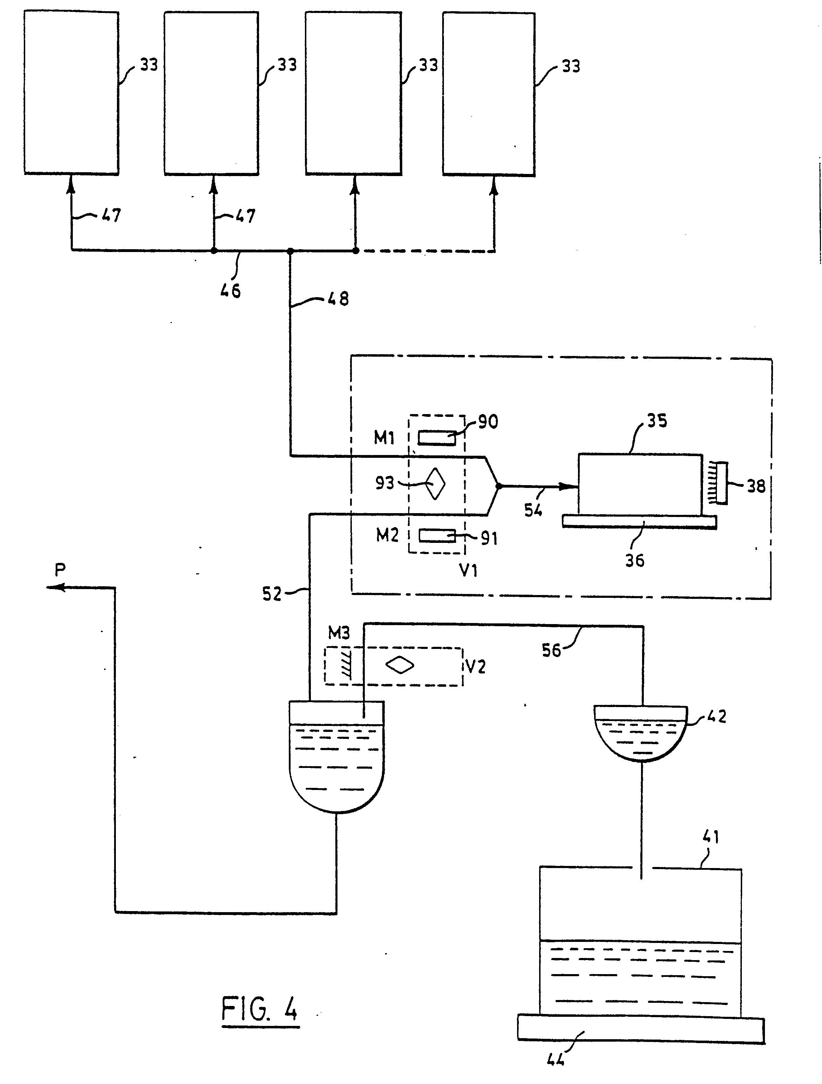

Attention is now directed to Figure 4, which illustrates the third embodiment of this invention.

In Figure 4, all portions which are the same as equivalent portions in Figure 2 bear the same numbers.

The only difference between the Figure 4 embodiment and the Figure 2 embodiment is the nature of the valves which are identified in Figure 4 as $M_1$, $M_2$ and $M_3$.

As can be seen in Figure 4, the composite valve $V_1$ consists of backing plates 90 and 91 in spaced-apart relation, and a reciprocating occluder member 93 which is adapted to move back and forth between the plates 90 and 91, either trapping the tube 52 against the backing plate 91, or trapping the tube 48 against the backing plate 90. Since the valves 1 and 2 in Figure 2, corresponding to valves $M_1$ and $M_2$ in Figure 4, are never both open or both closed at the same time, it is convenient to provide the special composite valve assembly $V_1$ shown in Figure 4.

A similar valve assembly, but working only on one side is identified as $V_2$ in Figure 4, and is adapted to open and close the valve $M_3$.

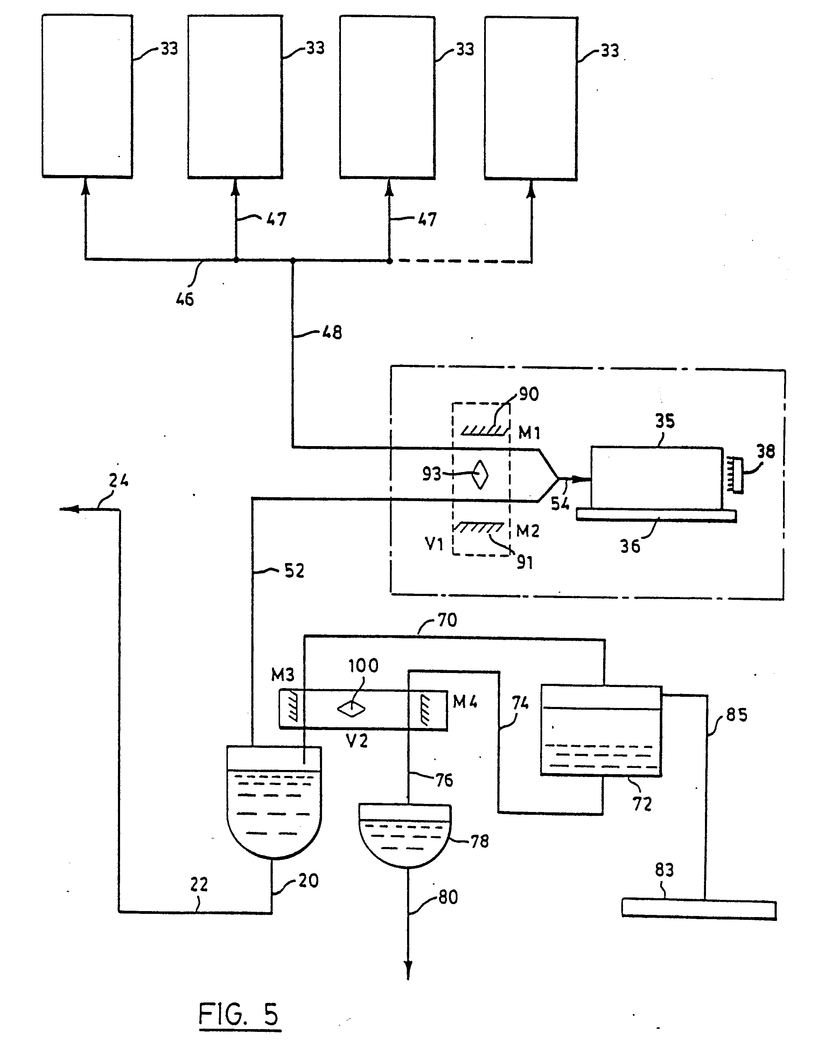

Attention is now directed to Figure 5, which is based on the second embodiment shown in Figure 3, and in which the equivalent portions and elements bear the same numbers. The only difference between the embodiment of Figure 5 and the embodiment of Figure 3 is the presence of the two valve assemblies $V_1$ and $V_2$. The Assembly $V_1$ operates in the same manner as just described in connection with Figure 4. The valve assembly $V_2$ has an occluder member 100 which alternately closes the valves $M_3$ and $M_4$, alternately trapping the tube 70 or the tube 74/76. Since these tubes are never both open or both closed at the same time, it is possible to use a valve assembly such as shown in Figure 5.

Returning now to the second embodiment of the invention shown in Figure 3, it is pointed out that this version is intended for operators who for personal reasons wish to use the waste bag 72. In this version, it is proposed that there be additional functional parameters in the DWELL mode. The timer/computer would keep track of fluid outflow from the waste bag 72 and would generate an alarm signal if such flow rate were below a particular threshold.

Turning to Figure 4, it is pointed out that there is considerable simplicity of the tubing arrangement, which leads to ease and simplicity of operational set-up for the sterile tubing.

In the fourth embodiment shown in Figure 5, and also in the second embodiment shown in Figure 3, it is contemplated that the timer/computer would generate an alarm signal if the outflow rate from the waste bag 72 is below a certain minimum.

For compactness of the embodiments of Figs. 3 and 5, it would be possible to install both of the scales in the same housing, for example the housing for the bag 35. The waste bag 72 would simply be suspended from its respective scale.

The four embodiments of this invention exhibit the following advantages for both C.C.P.D. and I.P.D.

(1) The FILL is on the basis of fluid weight, rather than the conventional preselected time period. This ensures that the desired amount of dialysate will flow into the peritoneal cavity of the patient during the FILL mode. The timer/computer will generate an alarm signal if the flow rate into the peritoneal cavity of the patient is below a certain minimum, and also when no more fresh dialysate is available from the containers 33 (end of treatment).

(2) This apparatus saves precious treatment time. The apparatus will switch to the DWELL mode as soon as the preselected fluid weight is taken in by the patient.

(3) The simplicity of the disposable sterile tubing results in easy and quick machine set-up and considerable financial savings. These result from the elimination of the usual heater bag, as well as both the waste bag and the drainage bags in the first and third embodiments.

(4) By monitoring the weight in the reusable container 41 or the waste bag 72, the apparatus allows a check of the rate of patient fluid drain, and it permits an alarm to be sounded if there is no fluid draining from the patient. This allows a constant monitoring of the state of the patient's catheter and/or the patient's physiological condition and well-being. The machine is also enabled to measure ultrafiltration.

(5) The computation and display of the ultrafiltration enables the patient's weight loss to be controlled and monitored from exchange to exchange, and allows better blood pressure control.

(6) The apparatus of this invention eliminates the separate heater box which normally holds a heater bag. A heater box is typically placed very high and demands that the suspension for the cold dialysate containers be raised on a movable suspender above the heater box for proper operation. The cold dialysate suspender for this invention is stationary and is located at an easy-to-reach level for the operator. Hence, the cart or stand is simple, of light weight and portable.

While particular embodiments of this invention have been illustrated in the accompanying drawings and described hereinabove, it will be apparent to those skilled in the art that changes and modifications may be made therein without departing from the essence of this invention, as set forth in the appended claims.

CLAIMS:

1.      A dialysis apparatus comprising:  first means for supplying dialysis fluid, second means for receiving and heating dialysis fluid, third means for receiving and weighing a quantity of dialysis fluid, a catheter, structure supporting said first means above said second means, said second means above said catheter, and said third means below said catheter, a first fluid flow path interconnecting said first and second means, a second fluid flow path interconnecting said second means and said catheter, a third fluid flow path interconnecting said catheter and said third means,

characterized in that,

the second means is also capable of weighing dialysis fluid, and the apparatus further includes valve means for selectively

(i) blocking said first and third paths while leaving said second path open,

(ii) blocking said second and third paths while leaving the first path open, and

(iii) blocking the second path while leaving the first and third paths open,

and timer/computer means for

(a) maintaining mode (i) until a preselected reduction in weight occurs at said second means,

(b) then switching to and maintaining mode (ii),

(c) then switching to and maintaining mode (iii).

2.      The invention claimed in claim 1, in which the timer/computer means is further adapted to (d) calculate and display an ultrafiltration quantity, if any, by computing the difference between said preselected reduction in weight at said second means during mode (i) and the weight of the quantity of fluid received at said third means during the next following mode (iii), and in which each of modes (ii) and (iii) is maintained for a preselected period of time.

3.        The invention claimed in claim 2, in which the timer/computer means is further adapted to (e) generate an alarm signal if the ultrafiltration calculated in (d) exceeds a predetermined amount, and has means for manually selecting said predetermined amount.

4.        The invention claimed in claim 1, claim 2 or claim 3, which further includes a fourth flow path for fluid draining from said third means, and in which the valve means opens the fourth path in modes (i) and (ii), and closes the fourth path in mode (iii).

5.        The invention claimed in claim 1, claim 2 or claim 3, in which the portion of the valve means which closes the first and second paths includes a simple reciprocating element which closes one of said first and second paths while leaving the other open.

6.        The invention claimed in claim 1, claim 2 or claim 3, which further includes a fourth flow path for fluid draining from said third means, and in which the valve means includes a single reciprocating element which opens one of the third and fourth paths while closing the other, the valve means opening the fourth path in modes (i) and (ii) and closing the fourth path in mode (iii).

7.        The invention claimed in claim 1, claim 2 or claim 3, in which the third flow path includes a drip chamber.

8.        The invention claimed in claim 1, claim 2 or claim 3, in which the second flow path includes a drip chamber.

9.        The invention claimed in claim 1, in which both the second and third flow paths include drip chambers.

10.        The invention claimed in claim 1, claim 2 or claim 9, in which the flow paths are defined by flexible tubing.

11.        The invention claimed in claim 1, claim 2 or claim 3, in which the timer/computer means generates alarm signals if, during mode (iii) the rate at which the weight of collected fluid at the third means increases is not above a predetermined threshold rate, or if, by the end of the second preselected period of time, the said preselected reduction in weight at said second means during mode (i) is greater than the weight of the total quantity of fluid collected at said third means during the next following mode (iii).

12.      The invention claimed in claim 1, claim 2 or claim 3, in which the timer/computer means generates an alarm signal if during mode (i) the rate of reduction of weight at said second means is below a predetermined threshold.

13.      A method of peritoneal dialysis utilizing a dialysis apparatus comprising first means for supplying dialysis fluid, second means for receiving and heating dialysis fluid, third means for receiving and weighing a quantity of dialysis fluid, a catheter connected to the peritoneal cavity of a patient, structure supporting said first means above said second means, said second means above said catheter and said third means below said catheter, means defining a first fluid flow path interconnecting said first and second means, means defining a second fluid flow path interconnecting said second means and said catheter, and means defining a third fluid flow path interconnecting said catheter and said third means,

characterized in that,

the second means is also adapted to weigh dialysis fluid, the method including the following steps in rotational sequence:

(i) blocking said first and third paths while leaving the second path open, until a preselected reduction in weight occurs at said second means, whereby a quantity of dialysis fluid corresponding to the reduction in weight passes by gravity along said second flow path into the peritoneal cavity of the patient,

(ii) blocking said second and third paths while leaving said first path open, whereby to initiate replenishment of fresh dialysis fluid in the second means from the first means along said first flow path, while maintaining dialysis fluid in the peritoneal cavity of the patient, and

(iii) blocking said second path while leaving the first and third paths open, whereby fresh dialysis fluid continues to pass along said first path from the first means to the second means, and simultaneously dialysis fluid from the peritoneal cavity of the patient drains along the catheter and said third path into said third means,

mode (i) constituting the fill mode for the patient, mode (ii) constituting the dwell mode for the patient, and mode (iii) constituting the drain mode for the patient.

14. The method claimed in claim 13, in which dialysis fluid in said second means is heated, and in which each of modes (ii) and (iii) is maintained for a preselected period of time.

15. The apparatus claimed in claim 1, claim 2 or claim 3, in which the said first means comprises a plurality of prepackaged, sterile bags of dialysis fluid, and in which the portion of the second means which receives the dialysis fluid is constituted by a further one of such prepackaged, sterile bags of dialysis fluid.

16. The method claimed in claim 13 or claim 14, which further includes using, as the said first means, a plurality of prepackaged, sterile bags of dialysis fluid, and using, as the portion of the second means which receives the dialysis fluid, a further one of such prepackaged, sterile bags of dialysis fluid.

17. A dialysis apparatus comprising: first means for supplying dialysis fluid, said first means comprising a plurality of prepackaged, sterile bags of dialysis fluid, second means for receiving and heating dialysis fluid, the portion of the second means which receives the dialysis fluid being constituted by a further one of such prepackaged, sterile bags of dialysis fluid, third means for receiving and weighing a quantity of dialysis fluid, a catheter, structure supporting said first means above

21                    0097432

said second means, said second means above said catheter
and said third means below said catheter, a first fluid
flow path interconnecting said first and second means,
a second fluid flow path interconnecting said second means
and said catheter, a third fluid flow path interconnecting
said catheter and said third means,

   characterized in that,

   said second means is also adapted to weigh
dialysis fluid, the apparatus further including valve
means for selectively

   (i)   blocking said first and third paths while
   leaving said second path open,
   (ii) blocking said second and third paths while
   leaving the first path open, and
   (iii) blocking the second path while leaving
   the first and third paths open,
   and timer/computer means for cycling modes (i),
(ii) and (iii) in sequence.

18.       A method of peritoneal dialysis utilizing a
dialysis apparatus comprising first means for supplying
dialysis fluid, said first means comprising a pluraltiy
of prepackaged, sterile bags of dialysis fluid, second
means for receiving and heating dialysis fluid, the
portion of said second means which receives the dialysis
fluid being a further one of such prepackaged, sterile
bags of dialysis fluid, third means for receiving and
weighing a quantity of dialysis fluid, a catheter
connected to the peritoneal cavity of a patient,
structure supporting said first means above said second
means, said second means above said catheter and said
third means below said catheter, means defining a first
fluid flow path interconnecting said first and second
means, means defining a second fluid flow path
interconnecting said second means and said catheter,
and means defining a third fluid flow path interconnecting
said catheter and said third means,

   characterized in that,

   said second means including means for weighing dialysis fluid in said further one of such prepackaged, sterile bags of dialysis fluid, the method including the following steps in rotational sequence:

(i) blocking said first and third paths while leaving the second path open, whereby a quantity of dialysis fluid passes by gravity along said second flow path into the peritoneal cavity of the patient,

(ii) blocking said second and third paths while leaving said first path open, whereby to initiate replenishment of fresh dialysis fluid in the second means from the first means along said first flow path, while maintaining dialysis fluid in the peritoneal cavity of the patient, and

(iii) blocking said second and fourth paths while leaving the first path open, whereby fresh dialysis fluid continues to pass along said first path from the first means to the second means, and simultaneously dialysis fluid from the peritoneal cavity of the patient drains along the catheter and said third path into said third means,

mode (i) constituting the FILL mode for the patient, mode (ii) constituting the DWELL mode for the patient, and mode (iii) constituting the DRAIN mode for the patient.

19. The apparatus claimed in claim 1 or claim 17, in which the weighing portions of said first and second means are mounted within a single housing, for compactness of the apparatus.

## FIG. 1
### PRIOR ART

FIG 2

FIG. 3

0097432

FIG. 4

FIG. 5